# EUROPEAN PATENT APPLICATION

(11) **EP 3 508 186 A1**
(43) Date of publication of application: **10.07.2019**
(21) Application number: 17794357.8
(22) Date of filing: 30.08.2017
(51) Int. Cl.: A61H 9/00, A61H 33/00, A47K 3/12

(54) **DEVICE FOR HYDROTHERAPY**

(30) Priority: 02.09.2016 ES 201631148
(71) Applicant: Mateos Aguilar, Richard, 03184 Torrevieja (ES)
(72) Inventor: Mateos Aguilar, Richard, 03184 Torrevieja (ES)
(74) Representative: Donoso Romero, José Luis
(86) International application number: PCT/ES2017/070590
(87) International publication number: WO 2018/042067

(57) **Abstract**

1.-Device (1) for hydrophysiotherapy comprising:
- a source of water supply (2),
- at least one driver and / or pressure regulator (5, 50) for supplying water at certain pressures from the water supply source (2),
-at least one hose (3, 4) connected to the driver and / or a pressure regulator (5, 50) and provided with a therapeutic nozzle (6, 7) with a handle (51) for manual operation, and
-a patient's aerial rest facilities (8).

## Description

### OBJECT OF THE INVENTION

This invention relates to a hydrophysiotherapy device.

### BACKGROUND OF THE INVENTION

At present, certain physiotherapy manoeuvres can be performed with the use of water, which improves muscular toning.

An example would be the pool of jets existing in spas and similar contexts, where the user is partially submerged in the pool and in the area of influence of a jet driver, so that it is the user who directs on which part of the body the jet acts by moving position. The disadvantage of this is that the user is often not clear where he or she has to direct the jet for proper effect or the correct massage, or he or she is not able to position it with precision. In addition, the jet, when submerged, is restricted and loses force.

Another example would be the Scottish shower - or shower of alternating hot and cold water - that dilates the blood vessels and shrinks them alternatively, reactivating the blood circulation and toning the skin: an effect known as hydrotherapy. However, it does not have appreciable muscular effects nor does it serve to treat specifically certain incidences such as contractures or muscular toning in general

### DESCRIPTION OF THE INVENTION

The configuration of the invention's hydrophysiotherapy device enables hydrotherapy and physiotherapy to be combined, since it allows muscular pressure manoeuvres to be applied on the patient by means of water jets easily managed by the professional physiotherapist and therefore directed with precision to the most effective areas in order to obtain the desired effects.

In accordance with the invention, the device comprises in its most basic form:
- a water supply source,
- at least one driver and / or pressure regulator to provide water at certain pressures from the water supply source,

- at least one hose connected to the driver and / or a pressure regulator, provided with a therapeutic nozzle with a handle for manual operation, and
- a patient's aerial rest facilities.

This allows the physiotherapist to handle the hose and direct the therapeutic nozzle(s) to the appropriate areas on the patient with the nozzle or hose handles, while the patient's aerial rest allows the jets to reach the desired area without any pause or interference. The driver and / or pressure regulator would be any feature pertaining to the state of the art that is capable of modulating a certain flow rate at a desired pressure from the water supply source, such as a boost pump if the source pressure is too low, or a reducer or pressure regulator if the source pressure is too high. The device can be installed, for example, in a spa, or on the beach, and if necessary fitted with a drain.

In this document, a therapeutic nozzle is understood as a nozzle forming at its outlet a jet with a suitable arc, shape and / or pressure to achieve adequate pressure or contact on the patient. Aerial supports are understood as a facility allowing the patient to take up a resting position, for example lying down, so that his or her muscles are relaxed as they would be if resting a physiotherapy table, and without the user being submerged, in order that the jets are not stopped by the immersion itself, and also leaving exposed the part of the musculature or anatomy of the patient on which the jets will be aimed.

### BRIEF DESCRIPTION OF THE DRAWINGS

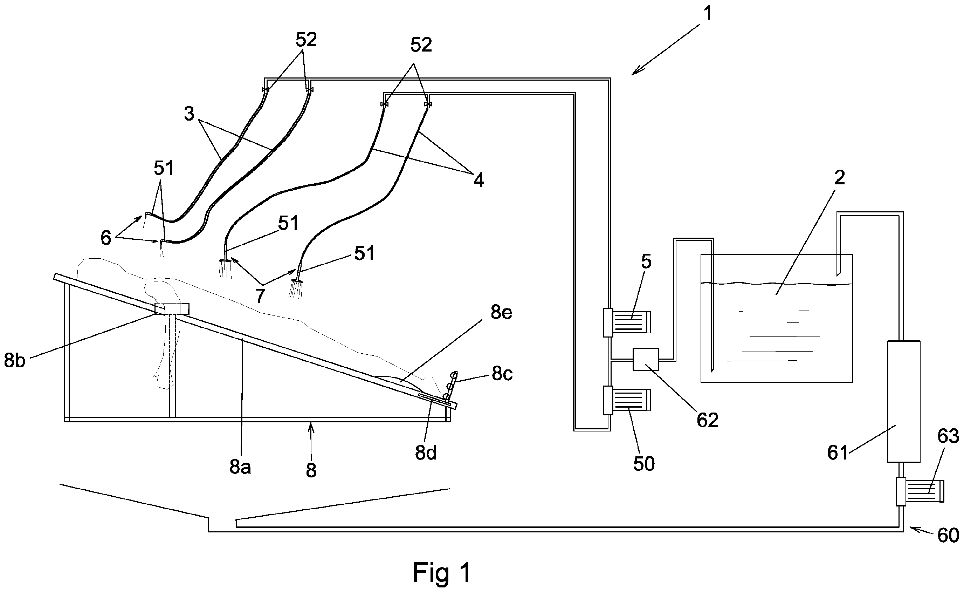

Figure 1 shows a schematic overview of the invention's device.
Figure 2 shows a detail in top view of the patient's overhead rest, comprising a bed.
Figures 3 and 4 show two details of two therapeutic nozzles of the invention's device.

### DESCRIPTION OF A PRACTICAL APPLICATION OF THE INVENTION

The hydrophysiotherapy device (1) of the invention comprises:
- a source of water supply (2),
- at least one driver and / or pressure regulator (5, 50) for supplying water at certain pressures from the water supply source (2),

- at least one hose (3, 4) connected to the driver and / or a pressure regulator (5, 50) and provided with a therapeutic nozzle (6, 7) with a handle (51) for manual operation, and
- a patient's aerial rest facilities (8).

Typically, the water supply source (2) would comprise a tank for sufficient quantity without having to depend on the pressure or flow rate of the network.

In this non-limiting example of the invention, the pressure driver and / or pressure regulator (5, 50) comprises at least one pump (motor pump) for expelling the water from the tank at the appropriate pressure.

Precisely as there are physiotherapy manoeuvres requiring greater or lesser pressure and active surface, it is anticipated that the device (1) should preferably comprise at least one first high flow and low and / or high-pressure pump (5) and a second low pressure, high pressure pump (50). For example, the first pump 5 would be a centrifugal pump of 2 bar and 15000 l / h and the second pump 50 would be a pressure pump of 6 bar and 50 I / min.

It is also envisaged that the device (1) ideally comprises two first hoses (3) connected to the first pump (5) and two second hoses (4) connected to the second pump (50), so that the physiotherapist is allowed to work with both hands with the same type of hoses (3, 4) simultaneously. The corresponding cut-off keys (52) are provided in the hoses (3, 4) to select the operating hoses. By means of a console (not shown), it is possible to control the start-up of the pumps, regulate the pressure of the jets and / or the opening or closing of the cut-off valves (52). In addition, in certain installations of a specific size, a water recovery circuit (60) may be provided with the appropriate purifiers (61) and pump features (63) where appropriate in order to disinfect the water before reuse, and / or heaters (62) for the water.

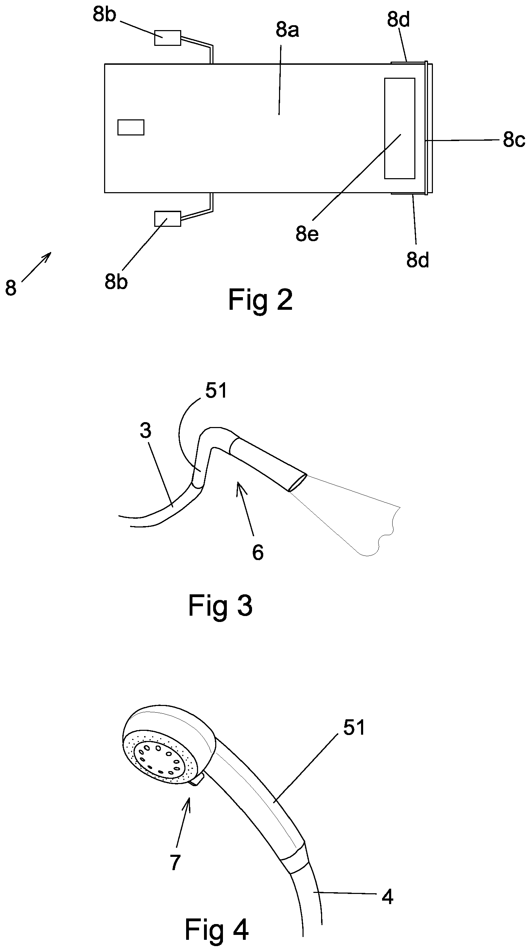

The therapeutic nozzles connected to the first hoses (3) would ideally comprise simple nozzles (6) (that is, with a single outlet having various shapes, such as pointed (see fig. 3), or round), in order to produce a low and / or high-pressure single, high-flow jet. The therapeutic nozzles connected to the second hoses would comprise spray nozzles (7) - that is, they would have diffusers or multiple outlets in order to provide have a high pressure multi jet. These spray nozzles (7) may for example comprise showerheads (single or adjustable flow) (see fig. 4).

In addition, the therapeutic nozzles (6, 7) are mounted on the respective hoses (3, 4) through interchangeable fittings, allowing different nozzles to be selected and used as required.

Finally, it should be noted that the patient's aerial rests (8) would preferably comprise a moisture resistant bed provided with a continuous support (8a) for the head and / or torso and / or lower limbs, and independent supports (8b) for the upper and / or lower limbs. It is also expected that they may have a footrest (8c) if they are titled in order to prevent the patient from slipping. The footrest (8c) will ideally have a lockable slider (8d) to adjust its position to the patient's height. A cushion (8e) for leg support will also be provided.

Obviously, the present non-restrictive example describes a device with a single bed although the service of more than one stretcher is possible; for example, in a spa or health resort, through more powerful pumps that supply several hoses
Having fully described the nature of the invention and the manner in which it is applied, it should be noted that the foregoing provisions and represented in the accompanying drawings are susceptible to modifications in detail insofar as they do not alter the fundamental principle.

## Claims

1. Device (1) for hydrophysiotherapy **characterised in that it** comprises:
- a water supply source (2),
- at least one driver and / or pressure regulator (5, 50) for supplying water at certain pressures from the water supply source (2),
- at least one hose (3, 4) connected to the driver and / or a pressure regulator (5, 50) and provided with a therapeutic nozzle (6, 7) with a handle (51) for manual operation, and
- a patient's aerial rest facilities (8).

2. Device (1) for hydrophysiotherapy according to claim 1 **characterised in that** the water supply (2) comprises a cistern.

3. Device (1) for hydrophysiotherapy according to any one of the preceding claims, **characterised in that** the driver and / or pressure regulator (5, 50) comprises at least one delivery pump.

4. Device for hydrophysiotherapy according to claim 3, **characterised in that** it comprises at least one first high-flow and low and/or high-pressure pump (50), and a second low-flow high pressure pump.

5. Device (1) for hydrophysiotherapy according to claim 4, **characterised in that** the first pump (5) is a 2 bar and 15000 l / h centrifugal pump.

6. Device (1) for hydrophysiotherapy according to any one of claims 4 or 5, **characterised in that** the second pump (50) is a 6 bar and 50 l / minute pressure pump.

7. Hydrophysiotherapy device (1) according to any one of claims 4 to 6, **characterised in that** it comprises two first hoses (3) connected to the first pump (5) and two second hoses (4) connected to the second pump (50).

8. Device (1) for hydrophysiotherapy according to any one of claims 4 to 7, **characterised in that** las the therapeutic nozzles connected to the first hoses comprise single nozzles (6).

9. Device (1) for hydrophysiotherapy according to any one of claims 4 to 8, **characterised in that** the therapeutic nozzles connected to the first hoses comprise spray nozzles (7).

10. Device (1) for hydrophysiotherapy according to claim 9 **characterised in that** the spray nozzles (7) comprise showerheads.

11. Device (1) for hydrophysiotherapy according to any one of the preceding claims, **characterised in that** the therapeutic nozzles (6, 7) are mounted on the respective hoses (3, 4) by means of interchangeable fittings.

12. Device (1) for hydrophysiotherapy according to any one of the preceding claims, **characterised in that** the hoses (3, 4) comprise cut-off valves (52).

13. Device (1) for hydrophysiotherapy according to claim 12 **characterised in that** it comprises a console for controlling the start-up of the pumps, regulating the pressure of the jets and / or the opening or closing of the cut-off valves (52).

14. Device (1) for hydrophysiotherapy according to any one of the preceding claims, **characterised in that** the aerial rest means (8) of the patient comprises a moisture resistant bed provided with a continuous support (8a) for the head and / or trunk and or lower limbs and independent supports (8b) for the upper and / or lower limbs.

15. Device (1) for hydrophysiotherapy according to claim 14 **characterised in that** the bed has a footrest (8c) featuring a lockable slider (8d) for adjusting its position to the height of the patient. It also comprises a cushion (8e) for leg rest.

16. Device (1) for hydrophysiotherapy according to any one of the preceding claims, **characterised in that** it includes a water recovery circuit (60) with purifiers (61).

17. Device (1) for hydrophysiotherapy according to any one of the preceding claims, **characterised in that** it comprises water heaters (62).
